# EUROPEAN PATENT SPECIFICATION

(11) **EP 4 108 180 B1**
(45) Date of publication and mention of the grant of the patent: **17.09.2025**
(21) Application number: 22180642.5
(22) Date of filing: 23.06.2022
(51) Int. Cl.: A61B 8/08, A61B 8/12, A61B 8/00, G01S 15/89

(54) **VISUALIZATION OF 4D ULTRASOUND MAPS**
VISUALISIERUNG VON 4D-ULTRASCHALLKARTEN
VISUALISATION DE CARTES 4D À ULTRASONS

(30) Priority: 24.06.2021 US 202117357303
(43) Date of publication of application: 28.12.2022
(73) Proprietor: Biosense Webster (Israel) Ltd., 2066717 Yokneam (IL)
(72) Inventor: ALTMANN, Andres Claudio, 2066717 Yokneam (IL); GOVARI, Assaf, 2066717 Yokneam (IL)
(74) Representative: Carpmaels & Ransford LLP

(56) References cited:
- US-A1- 2007 073 135
- US-A1- 2017 020 486
- US-B1- 11 039 883
- ETHAN BUNTING ET AL: "Cardiac Lesion Mapping in vivo using Intracardiac Myocardial Elastography", 1 January 2019 (2019-01-01), XP055745552, Retrieved from the Internet <URL:https://www.ncbi.nlm.nih.gov/pmc/articles/PMC5747324/pdf/nihms919938.pdf> [retrieved on 20201030], DOI: 10.1109/TUFFC.2017.2768301

## Description

### FIELD OF THE INVENTION

The present invention relates generally to medical visualizing methods, and particularly to visualizing ultrasound data acquired using an intra-body medical ultrasound probe.

### BACKGROUND OF THE INVENTION

US2017/020486A1 describes a method for acquiring 3D quantitative ultrasound elastography volumes. The method comprises using a 2D ultrasound transducer to scan a volume of tissue through which shear waves are created using an external vibration source, the synchronized measurement of tissue motion within the plane of the ultrasound transducer with the measurement of the transducer location in space, the reconstruction of tissue displacements in time and space over a volume from this synchronized measurement, and the computation of one or several mechanical properties of tissue from this volumetric measurement of displacements. US2007/073135A1 describes integrated ultrasound imaging and an ablation probe.

Various ultrasound visualization techniques to assess organ functioning within the body have been previously proposed in the patent literature. For example, U.S. Patent Application Publication 2020/0214662 describes systems and methods for generating an electromechanical map. The methods include obtaining ultrasound data comprising a series of consecutive image frames and radio frequency (RF) signals corresponding to a tissue location in the heart. Displacements and strains are measured based on the ultrasound data to determine an electromechanical activation in the location. The ultrasound data is converted into a series of isochrone maps, and the series of isochrone maps is combined, to generate the electromechanical map. The electromechanical map illustrates the electromechanical activation and internal wall structures of the heart.

As another example, U.S. Patent Application Publication 2010/0099991 describes an ultrasonic diagnostic imaging system that produces 3D images of blood flow which depict both the location of blood pools and flow velocity in one image. B mode data is acquired over a volumetric region and inverted to a range of grayscale value which highlights anechoic regions relative to regions of strong echo returns. Flow data is acquired over the same volumetric region and both data sets are volume rendered. The two volume renderings are then merged into a single 3D image in which the B mode pixel values are tinted in accordance with flow at the pixel locations.

U.S. Patent 8,090,429 describes systems and methods for co-registering, displaying and quantifying images from numerous different medical modalities, such as CT, MRI and SPECT. In this approach, co-registration and image fusion is based on multiple user-defined Regions-of-Interest (ROI), which may be subsets of entire image volumes, from multiple modalities, where each ROI may depict data from different image modalities. The user-selected ROI of a first image modality may be superposed over or blended with the corresponding ROI of a second image modality, and the entire second image may be displayed with either the superposed or blended ROI.

There remains a need however for improved means for simultaneously presenting a physician or operator with ultrasound images, such as 4D ultrasound images, and correlated visual data, such as electrophysiological data.

### SUMMARY OF THE INVENTION

The present invention provides medical systems as claimed hereinafter.

### Outline of the technology

Certain surgical methods are described with reference to the system of the present invention. Whilst no claim is directed to these methods *per se,* the system is capable of being used and is intended to be used in such methods. The present invention will be more fully understood from the following detailed description of the embodiments thereof, taken together with the drawings in which:

### BRIEF DESCRIPTION OF THE DRAWINGS

Fig. 1 is a schematic, pictorial illustration of a catheter-based ultrasound imaging system using a catheter with a distal end assembly comprising a 2D ultrasound-array and a location sensor, in accordance with an embodiment of the present invention;
Fig. 2 is a schematic, pictorial illustration of a visualization of a scarred tissue region in an inner wall section of an organ imaged using the ultrasound system of Fig. 1, in accordance with an embodiment of the present invention;
Fig. 3 is a schematic, pictorial illustration of ultrasound intracardiac acquisition using the system of Fig. 1, followed by derivation of a combined tissue motion map overlaying an endocardium image over a respective epicardium image;
Fig. 4 is a schematic, pictorial illustration showing the incorporation of an icon into a 3D rendering, the icon indicative of the type of ultrasound catheter used in generating the rendering;
Fig. 5 is a schematic, pictorial illustration of a graphical user interface (GUI) configured to generate and display a combined image made by weighting images from different imaging modalities;
Fig. 6 is a flow chart that schematically illustrates a method for deriving and displaying the results of Fig. 2;
Fig. 7 is a flow chart that schematically illustrates a method for deriving and displaying the results of Fig. 4;
Fig. 8 is a flow chart that schematically illustrates a method for showing the incorporation of an icon into a 3D rendering, where the icon is indicative of the type of ultrasound catheter used in generating the rendering; and
Fig. 9 is a flow chart that schematically illustrates a method for deriving and displaying the results of Fig. 5.

### DETAILED DESCRIPTION OF EMBODIMENTS

### OVERVIEW

Embodiments of the present disclosure that are described hereinafter provide methods and systems that use a probe, such as a catheter, having a two-dimensional (2D) array of ultrasound transducers for producing three-dimensional (3D) or four-dimensional (4D) ultrasound images. In the present context, the term "3D ultrasound image" refers to an ultrasound image that represents a certain volume in three dimensions. The term "4D ultrasound catheter" refers to a catheter incorporating a 2D array of ultrasound transducers. The term "4D ultrasound image" refers to a time series of 3D ultrasound images of a certain volume acquired by the 2D array. A 4D image can be regarded as a 3D movie, the fourth dimension being time. Another way of describing a 4D image (or rendering) is as a time-dependent 3D image (or rendering). Where used in the heart, a 4D ultrasound catheter may be referred to as "4D Intracardiac Echocardiography (ICE)" catheter.

In the disclosed embodiments, the catheter also comprises an integral location sensor, such as a magnetic position sensor, that is pre-registered with the 2D array. The 2D array produces a 3D sector-shaped ultrasound beam occupying a defined solid angle; (such a beam is referred to herein as a "wedge," as opposed to a 1D array "fan"). The 2D array is thus able to image a 2D section of an inner wall of an organ, such as of a cardiac chamber. Because of the integral location sensor, the spatial coordinates of every voxel in the imaged section is known based on the known relative position and orientation on the catheter shaft between the location sensor and the 2D array.

It may be possible to generate a 4D ultrasound rendering, and, at the same time, an electrophysiological (EP) rendering, of a portion of the heart. However, it is difficult for a physician to correlate the two separate renderings.

Some embodiments of the present invention overlay EP data, such as local activation times (LATs) or bipolar voltages, on a 4D ultrasound image or 3D rendering, the 3D/4D representation generated by a 4D ultrasound catheter. Because the 4D catheter has an integral location sensor and its pre-registration with the 2D array, the two entities, i.e., the EP values and the ultrasound image, are automatically registered in the combined image so that registration of the EP parameters with the ultrasound image is not required.

For example, the 4D ultrasound image may show 3D details of a wall of a heart chamber, as well as movement of the wall, so that the combined image shows these details as well as the electrical activity (e.g., the EP data). This will be apparent, for example, in a wall tissue region of a scar, where the mechanical movement of the tissue is different from that of surrounding unscarred tissue, and the electrical propagation in the region of the scar is different from that of unscarred tissue. The combined image may enhance a capability of a clinician to diagnose and decide on a treatment of a cardiac problem related to scars. In another embodiment, a user may import strain information from the ultrasound images and use a processor to compare and/or display the imported strain information with the electrical activity. This enables correlation between mechanical and electrical heart functionality.

Standard ultrasound images of surfaces of the walls of a heart chamber, i.e., the endocardium and the epicardium images, can be acquired. However, the different surfaces are hard to comprehend, even though the images are somewhat displaced in an ultrasound image, as both surfaces depicted in grayscale means it is impossible to clearly see the two surfaces. Typically, the endocardium and epicardium images are obtained by a processor segmenting the volume data acquired by the ultrasound probe. If an anatomical map exists, the processor may use the map to identify the different surfaces.

Some embodiments of the present disclosure allow a user of the 4D ultrasound catheter to apply different color palettes to the two images of the surfaces of the chamber wall. This renders the two surfaces clearly differentiable, by, for example, a processor overlaying a colored endocardium image on an epicardium image that is colored differently. The user may also adjust the transparency of the overlay to further enhance the combined image displayed.

As the ultrasound catheter is registered to the mapping location system, the system can use this pre-acquired knowledge to color the multiple structures (e.g., the endocardium and the epicardium) in the ultrasound image with different color scales.

Moreover, each area in each of the ultrasound images can be colored with a unique color scale according to its functionality, or used definition, such as using different color palette comprising, for example, warm colors or cold colors. For example, after mapping the right atrium, the ultrasound image area of the right atrium can be colored differently (with different color scales, such as red with different intensity according to the gray levels, or different color palettes, such as warm colors vs. cold colors for the endocardium and the epicardium images).

Icons for ultrasound catheters do not differentiate between the types of ultrasound catheter used. For example, in contrast to a 1D catheter which produces an ultrasound fan, 2D catheters produce an ultrasound wedge. In an embodiment, to inform a reviewer how ultrasound data was acquired, a processor selects a specific icon to incorporate into an existing 3D surface ultrasound rendering. In this embodiment the processor may display an icon of a 2D catheter producing 4D images, by incorporating a wedge icon in a 3D/4D rendering.

In many medical procedures, images having different functionalities may be acquired, e.g., using different imaging modalities, and, for comparison purposes, these images may be registered. Since they are registered, the images may be combined with each other so that the different elements on the images may be viewed simultaneously. However, each image typically carries much visual information, so that combining the images may quickly lead to information overload.

Some embodiments of the present disclosure provide a graphical user interface (GUI) that allows a reviewer to generate and adjust a combined image by weighting the contributions of the separate images to the combined image. In an example, three available images are to be considered that are all registered one with other (e.g., to a coordinate system of a tracking system): a computerized tomography (CT) image, a magnetic resonance imaging (MRI) image, and an ultrasound (US) image. Using the GUI to weight the CT, MRI and US images, the reviewer combines the three images into one. One disclosed image weighting model, which a GUI may apply using a processor, is based on the assumption that the images are positioned at the apices of a triangle, where the triangle is used as a weighting selector. To this end, the reviewer can move a cursor within the triangle to select the weight applied to each image.

Note that the different images to be weighted and combined do not necessarily have to be of different modalities. Rather, these images can be different representations of a tissue region acquired by a same medical modality. For example, an EP mapping modality can generate a first representation of LATs in a color map, and a second representation of a bipolar voltages map provided in gray scales. Such a combined EP representation may possess enhanced clinical significance.

### SYSTEM DESCRIPTION

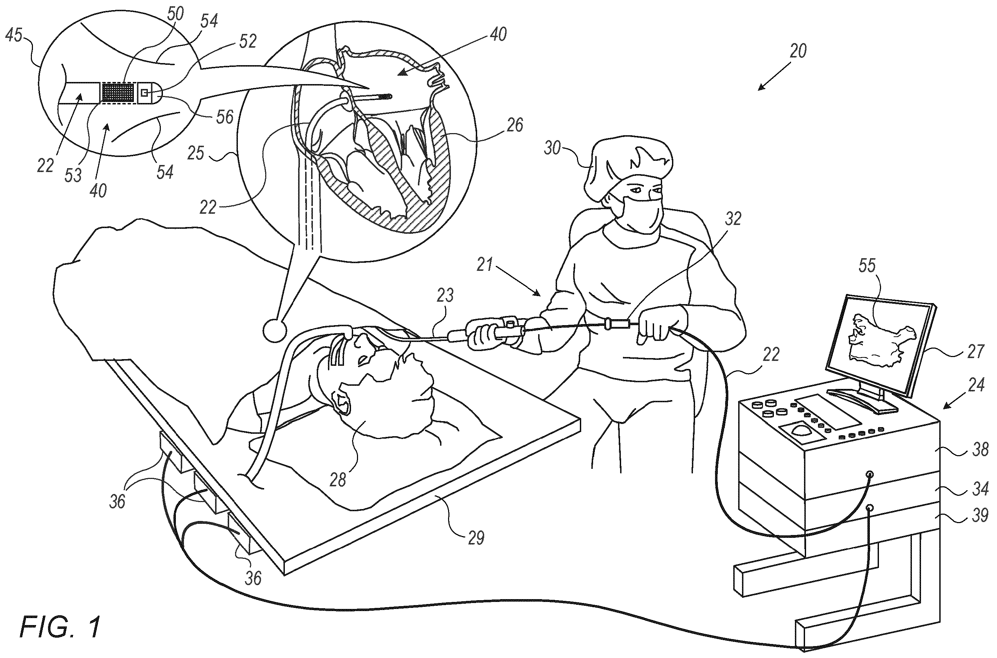

Fig. 1 is a schematic, pictorial illustration of a catheter-based ultrasound imaging system 20 using a catheter 21 with a distal end assembly 40 comprising a 2D ultrasound array 50 and a location sensor 52, in accordance with an embodiment of the present invention. Integral location sensor 52 is pre-registered with the 2D array 50 of catheter 21.

Specifically, sensor 52 is configured to output signals indicative of a position, direction and orientation of the 2D ultrasound transducer array 52 inside the organ. A processor of the system is configured to register multiple ultrasound image sections using the signal output by the sensor acquired by the 2D ultrasound transducer array 50, one with the other.

As seen, distal end assembly 40 is fitted at the distal end of a shaft 22 of the catheter. Catheter 21 is inserted through a sheath 23 into a heart 26 of a patient 28 lying on a surgical table 29. The proximal end of catheter 21 is connected to a control console 24. In the embodiment described herein, catheter 21 is used for ultrasound-based diagnostic purposes, although the catheter may be further used to perform therapy, such as electrical sensing and/or ablation of tissue in heart 26, using, for example, a tip electrode 56.

Physician 30 navigates distal end assembly 40 of catheter 21 to a target location in heart 26 by manipulating shaft 22 using a manipulator 32 near the proximal end of the catheter.

In an embodiment, 2D ultrasound-array 50, shown in detail in an inset 25, is configured to image a left atrium of heart 26.

As seen in an inset 45, ultrasound array 50 comprises a 2D array 50 of multiple ultrasound transducers 53. Inset 45 shows ultrasound array 50 navigated to an ostium 54 of a pulmonary vein of the left atrium. In this embodiment, 2D array 50 is an array of 32 × 64 US transducers. The 2D array is able to image a section of the inner wall of the ostium. Because of the integral location sensor, and its pre-registration with the 2D array, the spatial coordinates of every pixel in the imaged section are known by the system. An example of a suitable 2D array is described in D. Wildes et al., "4-D ICE: A 2-D Array Transducer With Integrated ASIC in a 10-Fr Catheter for Real-Time 3-D Intracardiac Echocardiography," in IEEE Transactions on Ultrasonics, Ferroelectrics, and Frequency Control, vol. 63, no. 12, pp. 2159-2173, Dec. 2016, doi: 10.1109/TUFFC.2016.2615602.

Control console 24 comprises a processor 39, typically a general-purpose computer, with suitable front end and interface circuits 38 for receiving signals from catheter 21, as well as for, optionally, applying treatment via catheter 21 in heart 26 and for controlling the other components of system 20. Console 24 also comprises a driver circuit 34, configured to drive magnetic field generators 36.

During the navigation of distal end 22 in heart 26, console 24 receives position and direction signals from location sensor 52 in response to magnetic fields from external field generators 36. Magnetic field generators 36 are placed at known positions external to patient 28, e.g., below table 29 upon which the patient is lying. These position and direction signals are indicative of the position and direction of 2D ultrasound-array 50 in a coordinate system of the position tracking system.

The method of position and direction sensing using external magnetic fields is implemented in various medical applications, for example, in the CARTO^{™} system, produced by Biosense Webster, and is described in detail in U.S. Patents 6,618,612 and 6,332,089, in PCT Patent Publication WO 96/05768, and in U.S. Patent Application Publications 2002/0065455, 2003/0120150, and 2004/0068178.

Exemplary catheters and imaging assemblies that enable deflection and rotation are described in detail in U.S. Patents 9,980,786; 10,537,306; and U.S. Patent Publication No. 2020-0061340 A1.

In some embodiments, processor 39 may be configured to operate array 52 in a "sweeping mode" to image a whole cardiac camber, as described below. In an embodiment, the imaged cardiac chamber (e.g., a left atrium) is presented to physician 30 by processor 39 on a monitor 27, e.g., in as a volume rendering 55.

Processor 39 typically comprises a general-purpose computer, which is programmed in software to carry out the functions described herein. The software may be downloaded to the computer in electronic form, over a network, for example, or it may, alternatively or additionally, be provided and/or stored on non-transitory tangible media, such as magnetic, optical, or electronic memory.

The example configuration shown in Fig. 1 is chosen purely for the sake of conceptual clarity. The disclosed techniques may similarly be applied using other system components and settings. For example, system 20 may comprise additional components and perform non-cardiac catheterizations.

### RENDERING AND VISUALIZATION OF 4D ULTRASOUND MAPS

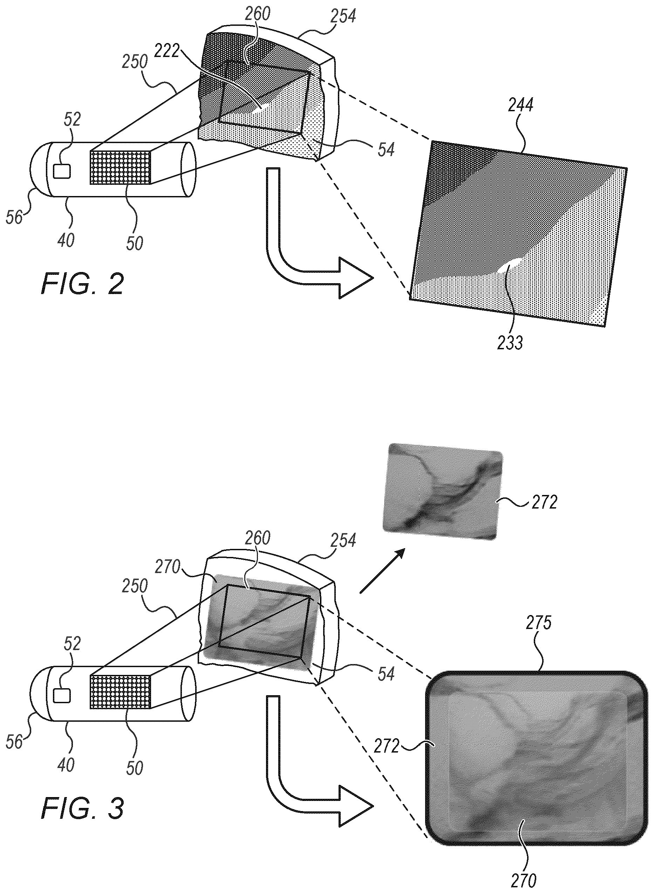

Fig. 2 is a schematic, pictorial illustration of a visualization of a scarred tissue region 222 in an inner wall 54 section 260 of an organ 254 imaged using ultrasound system 20 of Fig. 1, in accordance with an embodiment of the present invention. The acquisition is performed using catheter 21 of system 20 of Fig. 1. As seen, a 3D wedge 250 mode of acquisition enables simultaneous acquisition of the 2D section 260. Using location sensor 52, the geometry of ultrasound wedge 250 can be defined in a coordinate system of the location tracking system of system 20.

Using the registration, processor 39 derives a tissue color-coded motion map 244 from the acquisition, comprising a color-coded visualized scarred region 233. The processor distinguishes a scar by estimating that the mechanical movement of the tissue therein is different (typically lower or absent) from that of surrounding non-scarred tissue. In an embodiment, the processor graphically encodes (e.g., marks, or color codes), also in real time, e.g., on the video image that is displayed, the amounts of motion, to distinguish immobile areas that may be scarred tissue.

In an embodiment, the processor is configured to identify scarred tissue by comparing the level of tissue motion to a threshold value of expected amount of motion of a healthy tissue.

Fig. 3 is a schematic, pictorial illustration of ultrasound intracardiac acquisition using system 20 of Fig. 1, followed by derivation of a combined tissue motion map 275 overlaying an endocardium image 270 and a respective epicardium 272 image, in accordance with embodiments of the present invention.

In an embodiment, using location information from sensor 52, endocardium image 270 and its respective epicardium image 272 are extracted from an ultrasound acquisition/conventional image (e.g., the cardiac surfaces shown by images 270 and 272 are isolated from one another using an algorithm). Consider, for example, a scenario in which the catheter is in a cardiac chamber, or a user has selected a point in the chamber, or an anatomical Carto map already exists, and its center mass can be used. In an embodiment, the processor may apply an algorithm that extends a simulated beam ray from the selected point, such that the ray intersects the first surface and then the second border. Existing algorithms that are used today for segmentation of CT and MRI 3D scans can be also utilized, for example multi-thresholding based on statistical local and global parameters, mathematical morphology, and image filtering.

Subsequently, imaged section 360 is color coded in each of the ultrasound images with a unique color scale according to its functionality (e.g., amount of movement). The color code can be correlated to velocity, strain, voltage, thickness, local activation time and/or using another definition, for example by using different color scales, e.g., warm colors vs. cold colors.

In the shown example, the user may also adjust the transparency of the overlay 270 to further enhance the combined displayed image.

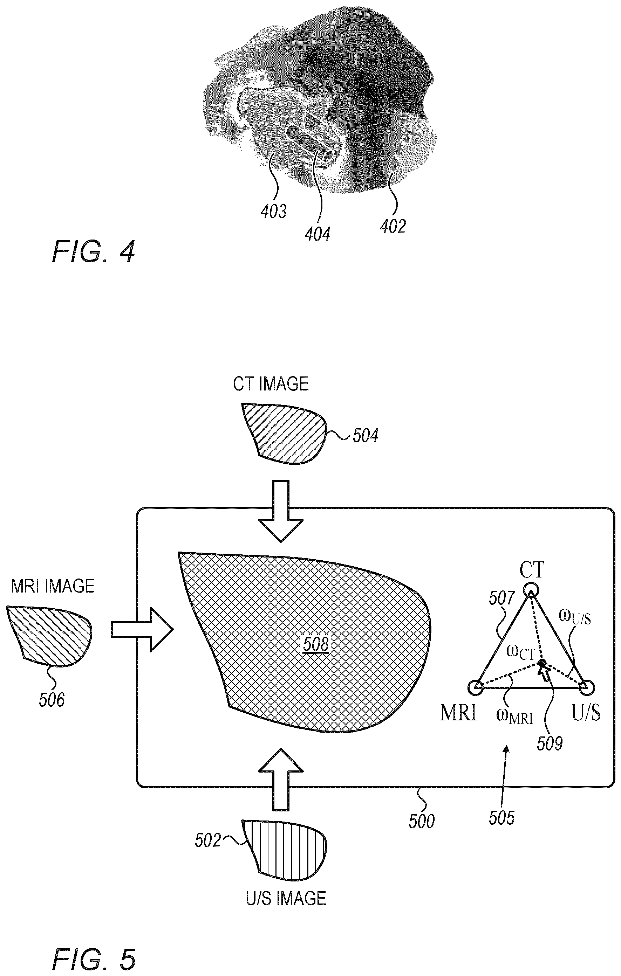

Fig. 4 is a schematic, pictorial illustration showing the incorporation of an icon 402 into a 3D rendering 404, where icon 402 is indicative of the type of ultrasound catheter used to generate the rendering, in accordance with embodiments of the present disclosure. Specifically, Fig. 4 shows a 3D rendering of a cardiac chamber 402, derived using system 20 of Fig. 1, that incorporates an icon 404 indicative of the type of ultrasound catheter 21.

In the shown embodiment, a region 403 of the surface rendering is made transparent by processor 39, which further incorporates therein icon 404, to show a wedge beam of 2D catheter 21 that was used for the intracardiac acquisition.

Fig. 5 is a schematic, pictorial illustration of a graphical user interface (GUI) 500 configured to generate and display a combined image 508 made by weighting images (502, 504 and 506) from different imaging modalities, in accordance with embodiments of the present disclosure. In other embodiments, the different images do not necessarily have to all be from different imaging modalities. For example, GUI 500 may combine EP maps with an image of an imaging modality combining an LAT map, bipolar voltage map and an ultrasound rendering.

In the shown embodiment, GUI 500 allows a reviewer of combined image 508 to select the display weighting that is applied to three images, a computerized tomography (CT) image 504, a magnetic resonance imaging (MRI) 506, and an ultrasound (US) image 502 that are all registered one with the other.

A disclosed image weighting algorithm 505 used with GUI 500 assumes the images are positioned at the apices of a triangle 507, and a reviewer can move a cursor 509 within the triangle area to select the relative weights ω applied to each image. In one embodiment the weights are normalized by the sum of weights that is kept equal to one, ω_{CT} + ω_{MRI} + ω_{U/S}=1. In another embodiment, the weights are normalized by the sum of squares of the weights and kept equal to one, ω²_{CT} + ω²_{MRI} + ω²_{U/S}=1.

GUI 500 and method of weighting using a triangle area are shown by way of example. Other implementations are possible, for example, using a circle or a polygon to represent the image-weighting space.

### METHODS OF VISUALIZATION OF 4D ULTRASOUND MAPS

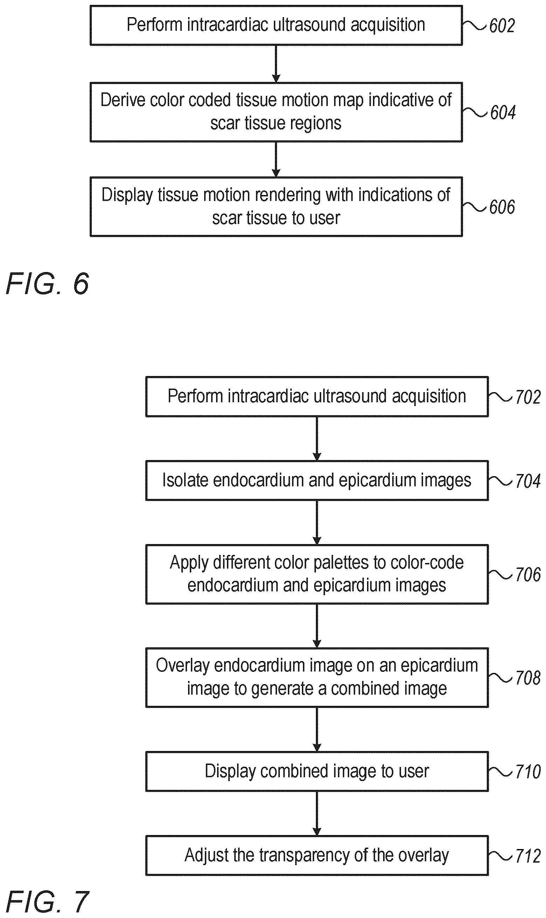

Fig. 6 is a flow chart that schematically illustrates a method for deriving and displaying the results of Fig. 2, in accordance with an embodiment of the present disclosure. The procedure begins by performing an ultrasound acquisition inside a cardiac chamber, such as shown in Fig. 1, at a 4D ultrasound acquisition step 602.

Next, at a tissue motion map derivation step 604, processor 39 derives a color-coded tissue-motion map that is indicative of scarred tissue regions, such as rendering 244 described in Fig. 2. To this end, the generated map of the tissue region is indicative of respective amounts of motion of tissue locations in the tissue region. /the processor identifies, based on the amounts of motion of the tissue locations, that tissue at one or more of the tissue locations comprises scar tissue.

At a rendering displaying step 606, processor 39 displays a cardiac tissue motion rendering of step 602 to a user, such as shown with rendering 55 on monitor 27 of Fig. 1. The presentation may include adding graphical indication, e.g., by coloring or patterning on map the scarred area, or by placing visual markings, to scarred regions encoded in rendering 244.

The flow chart of Fig. 6 is brought purely by example. In other embodiments, as another example, processor 39 performs pattern coding instead of color coding.

Fig. 7 is a flow chart that schematically illustrates a method for deriving and displaying the results of Fig. 4, in accordance with an embodiment of the present disclosure. The procedure begins by performing an ultrasound acquisition inside a cardiac chamber, such as shown in Fig. 1, at a 4D ultrasound acquisition step 702.

Next, processor 39 isolates endocardium and respective epicardium images of a same imaged section, such as images 270 and 272 of imaged section 360, shown in Fig. 3, at surfaces image extraction step 704.

In a color-coding step 706, processor 39 applies different color palettes (e.g., red-based and blue-based) to images 270 and 272, for example, to indicate amounts of motion.

At image overlaying step 708, processor 39 overlays endocardium image 270 on epicardium image 272 to generate a combined image 275.

Processor 39 displays combined image 275 to a user at a displaying step 710. Finally, at an adjustment step 712, the user adjusts a level of transparency of image 270 to make combined image 275 more visible.

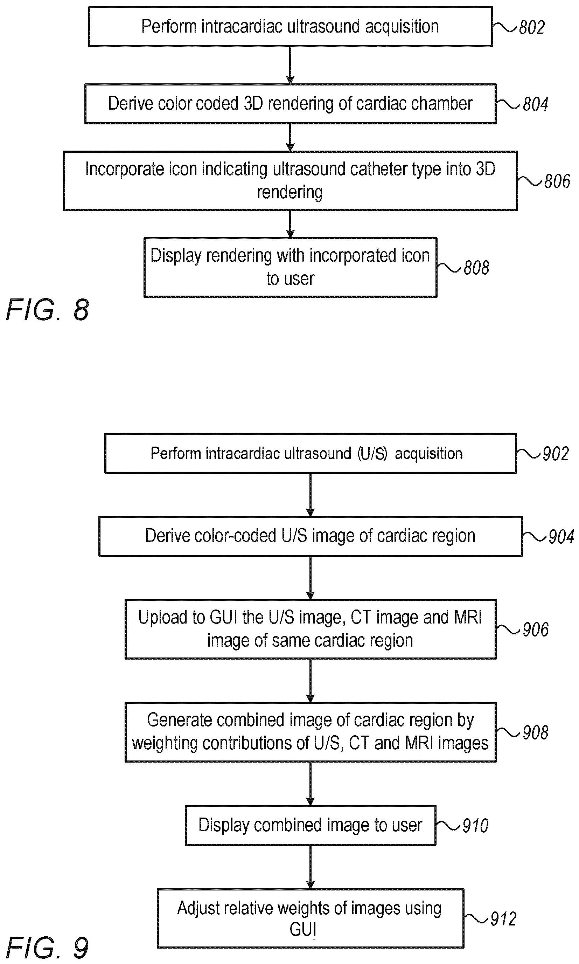

Fig. 8 is a flow chart that schematically illustrates a method for showing the incorporation of icon 404 into a 3D rendering 402, where the icon is indicative of the type of ultrasound catheter 21 used in generating the rendering, in accordance with embodiments of the present invention. The procedure begins by performing an ultrasound acquisition inside a cardiac chamber, such as shown in Fig. 1, at a 4D ultrasound acquisition step 802.

Next, processor 39 derives color coded 3D rendering of cardiac chamber 402, shown in Fig. 4, at a 3D rendering step 804.

At icon incorporation step 806, processor 39 incorporates icon 404 that shows a wedge ultrasound beam, indicating that a 2D ultrasound catheter, such as catheter 21, was used for acquiring the data at step 802.

Finally, at a displaying step 808, processor 39 displays the icon-incorporated rendering to a user.

Fig. 9 is a flow chart that schematically illustrates a method for deriving and displaying the results of Fig. 5, in accordance with an embodiment of the present invention. The procedure begins by performing an ultrasound (US) acquisition of a cardiac region, such as inside a cardiac chamber, such as shown in Fig. 1, at a 4D ultrasound acquisition step 902.

Next, processor 39 derives a color-coded US image, such as image 502, of a cardiac region, at an ultrasound imaging step 904.

At images uploading step 906, processor 39 uploads to GUI 500 US image 502, CT image 504, and MRI image 506. The images comprise a same cardiac region and are registered one with other by processor 39.

At a combined image generation step 908, processor 39 generates combined image 508 by weighing US image 502, CT image 504, and MRI image 506, using one of the normalizing weighting methods described above.

At a displaying step 910, processor 39 displays combined image 508 to a user.

Finally, at an adjustment step 912, the user adjusts the relative weights (e.g., contributions) of images 502 using GUI 500, 504, and 506, ω_{U/S}, ω_{CT}, and ω_{MRI}, respectively, by moving a cursor in GUI 500, as described above, to make combined image 508 more informative.

In embodiments described herein, processors of medical systems receive, from an ultrasound probe that may be provided separately to the medical systems, ultrasound images and signals indicative of respective positions, directions and orientations of a 2D ultrasound transducer array, and process them for various purposes.

Although the embodiments described herein mainly address cardiac applications, the methods and systems described herein can also be used in other body organs. For example, the disclosed technique can be used with transesophageal ultrasound (TEE) devices visualizing the heart. As another example, the disclosed technique may be used for invasive ultrasound lung imaging, and for visualizing liver and kidney.

It will thus be appreciated that the embodiments described above are cited by way of example, and that the present invention, which is defined in claim 1, is not limited to what has been particularly shown and described hereinabove. Rather, the scope of the present invention may include both combinations and sub-combinations of the various features described hereinabove, as well as variations and modifications thereof which fall within the scope of the appended claims.

## Claims

1. A medical system (20), comprising:
a processor (39), which is configured to:
receive, from an ultrasound probe (21), ultrasound images and signals indicative of respective positions, directions and orientations of a two-dimensional (2D) ultrasound transducer array (50);
using the received signals, register multiple ultrasound images of a tissue region (260), acquired over a given time duration by the 2D ultrasound transducer array, with one another;
generate a map (244) of the tissue region indicative of respective amounts of motion of tissue locations in the tissue region;
identify, based on the amounts of motion of the tissue locations, that tissue at one or more of the tissue locations comprises scar tissue (222); and
present the map to a user, with an indication (233) of the identified scar tissue.

2. The medical system according to claim 1, wherein the processor is configured to indicate the identified scar tissue using a graphically encoded level of motion of the tissue.

3. The medical system according to claim 2, wherein the graphically encoded level of motion comprises a color-coded level of motion.

4. The medical system according to claim 1, wherein the processor is configured to identify the scar tissue by comparing the amounts of tissue motion to a threshold value.

5. The medical system of any of claims 1 to 4, further comprising an ultrasound probe for insertion into an organ of a body, the ultrasound probe comprising:
a two-dimensional (2D) ultrasound transducer array; and
a sensor (52) configured to output signals indicative of a position, direction and orientation of the 2D ultrasound transducer array inside the organ.

## Patentansprüche

1. Medizinisches System (20), umfassend:
einen Prozessor (39), der konfiguriert ist zum:
Empfangen von Ultraschallbildern und -signalen von einer Ultraschallsonde (21), die die jeweiligen Positionen, Richtungen und Orientierungen eines zweidimensionalen (2D) Ultraschallwandler-Arrays (50) anzeigen;
Registrieren mehrerer Ultraschallbilder einer Geweberegion (260), die über einen bestimmten Zeitraum von dem 2D-Ultraschallwandler-Array aufgenommen wurden, miteinander unter Verwendung der empfangenen Signale;
Erzeugen einer Karte (244) der Geweberegion, die die jeweiligen Bewegungsgrade von Gewebestellen in der Geweberegion anzeigt;
Identifizieren, basierend auf dem Ausmaß der Bewegung der Gewebestellen, dass das Gewebe an einer oder mehreren der Gewebestellen Narbengewebe (222) umfasst; und
Präsentieren der Karte einem Benutzer mit einer Angabe (233) des identifizierten Narbengewebes.

2. Medizinisches System nach Anspruch 1, wobei der Prozessor konfiguriert ist zum Anzeigen des identifizierten Narbengewebes unter Verwendung eines grafisch kodierten Bewegungsgrades des Gewebes.

3. Medizinisches System nach Anspruch 2, wobei der grafisch kodierte Bewegungsgrad einen farbkodierten Bewegungsgrad umfasst.

4. Medizinisches System nach Anspruch 1, wobei der Prozessor konfiguriert ist zum Identifizieren des Narbengewebes durch Vergleichen des Ausmaßes der Gewebebewegung mit einem Schwellenwert.

5. Medizinisches System nach einem der Ansprüche 1 bis 4, ferner umfassend eine Ultraschallsonde zum Einführen in ein Organ eines Körpers, die Ultraschallsonde umfassend:
ein zweidimensionales Ultraschallwandler-Array (2D-Ultraschallwandler-Array); und
einen Sensor (52), der konfiguriert ist, um Signale auszugeben, die eine Position, Richtung und Ausrichtung des 2D-Ultraschallwandler-Arrays innerhalb des Organs anzeigen.

## Revendications

1. Système médical (20), comprenant :
un processeur (39), qui est configuré pour :
recevoir, d'une sonde à ultrasons (21), des images et des signaux ultrasonores indiquant les positions, directions et orientations respectives d'un réseau bidimensionnel (2D) de transducteurs ultrasonores (50) ;
à l'aide des signaux délivrés en sortie, enregistrer les unes par rapport aux autres plusieurs images ultrasonores d'une région tissulaire (260), acquises sur une durée donnée par le réseau de transducteurs ultrasonores 2D ;
générer une carte (244) de la région tissulaire indiquant les quantités respectives de mouvement des emplacements tissulaires dans la région tissulaire ;
identifier, sur la base de l'amplitude du mouvement des localisations tissulaires, que le tissu à une ou plusieurs des localisations tissulaires comprend du tissu cicatriciel (222) ; et
présenter la carte à un utilisateur, avec une indication (233) du tissu cicatriciel identifié.

2. Système médical selon la revendication 1, dans lequel le processeur est configuré pour indiquer le tissu cicatriciel identifié à l'aide d'un niveau de mouvement du tissu codé graphiquement.

3. Système médical selon la revendication 2, dans lequel le niveau de mouvement codé graphiquement comprend un niveau de mouvement codé en couleur.

4. Système médical selon la revendication 1, dans lequel le processeur est configuré pour identifier le tissu cicatriciel en comparant les quantités de mouvement du tissu à une valeur seuil.

5. Système médical selon l'une quelconque des revendications 1 à 4 comprenant en outre une sonde à ultrasons destinée à être insérée dans un organe d'un corps, la sonde à ultrasons comprenant :
un réseau bidimensionnel (2D) de transducteurs à ultrasons ; et
un capteur (52) configuré pour délivrer en sortie des signaux indiquant une position, direction et orientation du réseau de transducteurs à ultrasons 2D à l'intérieur de l'organe ;
